# FASCICULE DE BREVET EUROPEEN

(11) **EP 0 574 318 B1**
(45) Date de publication et mention de la délivrance du brevet: **17.04.1996**
(21) Numéro de dépôt: 93401482.0
(22) Date de dépôt: 10.06.1993
(51) Int. Cl.: C07J 71/00, C07J 5/00

(54) **Nouveau procédé de préparation de stéroides 16 alpha-méthylés**
Neues Verfahren zur Herstelling von 16-alpha-methyl-steroide
New process for the preparation of 16-alpha-methyl-steroids

(30) Priorité: 11.06.1992 FR 9207048
(43) Date de publication de la demande: 15.12.1993
(73) Titulaire: ROUSSEL UCLAF, F-93230 Romainville (FR)
(72) Inventeur: Buendia, Jean, F-94170 Le Perreux Sur Marne (FR); Roussel, Patrick, F-94320 Thiais (FR); Vivat, Michel, F-77400 Lagny Sur Marne (FR)
(74) Mandataire: Vieillefosse, Jean-Claude

(56) Documents cités:
- WO-A-87/07612
- DE-A- 2 407 967
- GB-A- 2 001 990
- US-A- 4 530 795
- JOURNAL OF THE CHEMICAL SOCIETY, 1961, Letchworth (GB); J. ATTENBURROW et al., pp. 4547-4559

## Description

La présente invention concerne un nouveau procédé de préparation de stéroïdes 16α-méthylés.

L'invention a ainsi pour objet un procédé de préparation des composés de formule (I): 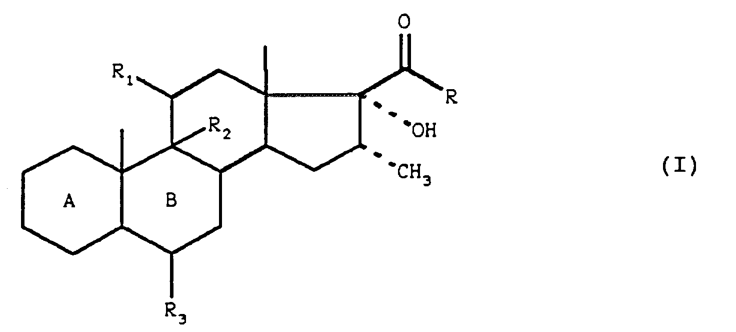 dans laquelle les cycles A et B représentent un reste : 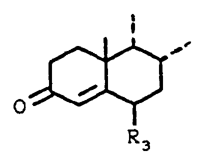 dans lequel la fonction cétone en position 3 est éventuellement protégée sous forme de cétal, de thiocétal, d'hémithiocétal ou d'éther d'énol, ou un reste : 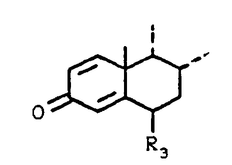 R représente un radical méthyle ou un radical -CH₂-OR', dans lequel R' représente un atome d'hydrogène ou un reste éther ou un reste ester, R₁ et R₂ forment ensemble une seconde liaison, ou R₁ et R₂ forment ensemble un époxyde en position β, ou R₁ représente un atome d'hydrogène, une fonction cétone ou une fonction hydroxy en position α ou β, libre ou protégée sous forme d'éther ou d'ester et R₂ représente un atome d'hydrogène, ou R₁ représente un atome d'hydrogène et R₂ représente une fonction hydroxy en position α, ou R₁ représente une fonction hydroxy en position β, libre ou protégée sous forme d'éther ou d'ester et R₂ représente un atome de fluor ou de brome en position α, et R₃ représente un atome d'hydrogène ou un atome de fluor ou un radical méthyle, en position α ou β, caractérisé en ce que l'on traite un composé de formule (II) : 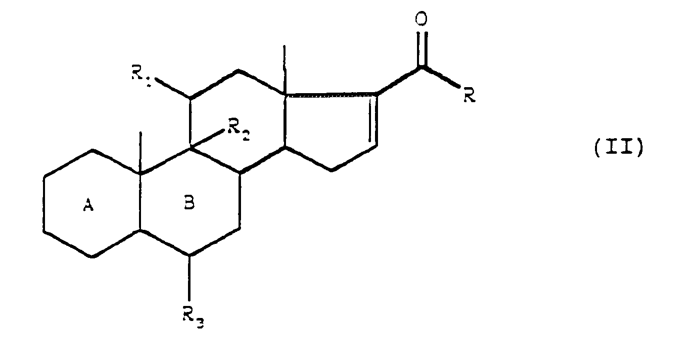 dans laquelle A, B, R, R₁, R₂ et R₃ ont la signification déjà indiquée, par un agent de méthylation en présence d'un catalyseur à base de cuivre, puis hydrolyse l'énolate méthylé pour obtenir l'énol correspondant que l'on oxyde par barbotage d'oxygène ou d'air dans le milieu d'hydrolyse en présence d'un agent réducteur complexant du cuivre, et compatible avec l'oxydant, pour obtenir le composé attendu.

Lorsque la fonction cétone en 3 est protégée sous forme de cétal, thiocétal ou hémithiocétal, il s'agit de préférence d'un groupement de formule : 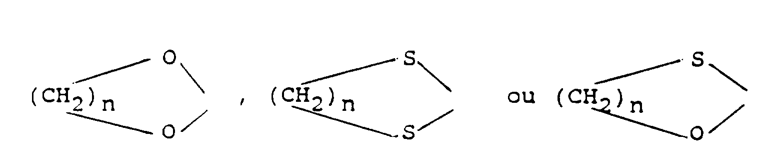 dans lequel n est égal à 2 ou 3 et tout particulièrement d'un groupement éthylènedioxy ou éthylènedithio.

Lorsque la fonction cétone en 3 est protégée sous forme d'éther d'énol, il s'agit de préférence d'un groupement alkoxy ou alkoxyalkoxy renfermant de 1 à 8 atomes de carbone et plus particulièrement d'un groupement méthoxy, éthoxy, éthoxyéthoxy ou 1-éthoxyéthoxy, les cycles A et B comportant alors un système de doubles liaisons Δ 3,5.

Lorsque R' représente un reste éther, il peut s'agir de tout reste connu de l'homme du métier et notamment d'un radical alkyle renfermant de 1 à 6 atomes de carbone, par exemple méthyle, éthyle ou propyle, d'un radical tétrahydropyranyle, ou d'un reste d'éther silylé, par exemple trialkylsilyle tel que triméthyl ou diméthylterbutylsilyle, triarylsilyle tel que triphénylsilyle ou diarylalkylsilyle tel que diphénylterbutylsilyle.

Lorsque R' représente un reste ester, il peut s'agir de tout reste connu de l'homme du métier et notamment d'un radical acyle renfermant de 1 à 8 atomes de carbone, par exemple formyle, acétyle, propionyle, butyryle, valéryle ou benzoyle.

Lorsque R₁ représente une fonction hydroxy protégée sous forme d'éther ou d'ester, il s'agit de l'un des restes éthers ou esters mentionnés plus haut pour R', étant entendu que ces restes ne sont pas nécessairement identiques.

L'invention a notamment pour objet un procédé tel que défini précédemment, caractérisé en ce que l'on utilise au départ un composé de formule (II) dans laquelle les cycles A et B représentent un reste : 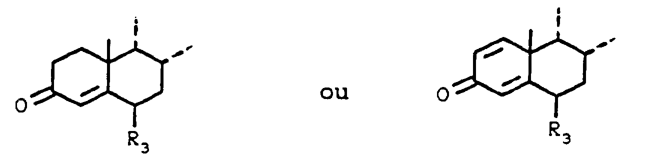 dans lequel R₃ est défini comme précédemment et la fonction cétone en position 3 est libre.

L'invention a aussi notamment pour objet un procédé tel que défini précédemment, caractérisé en ce que l'on utilise au départ un composé de formule (II) dans laquele R₁ et R₂ forment ensemble une seconde liaison, ou R₁ et R₂ forment ensemble un époxyde en position β, ou R₁ représente une fonction hydroxy en position β, libre ou protégée sous forme d'éther ou d'ester et R₂ représente un atome de fluor en position α, et R₃ représente un atome d'hydrogène, un atome de fluor ou un radical méthyle et, de préférence, un atome d'hydrogène.

L'agent de méthylation utilisé peut être un dérivé méthylé du cuivre, par exemple CH₃Cu, (CH₃)₂ CuMg, (CH₃)₂ CuLi ou, de préférence, un chlorure, bromure ou iodure de méthylmagnésium, utilisé en présence d'un catalyseur à base de cuivre. Le catalyseur peut être un sel tel que l'acétate, le propionate ou le chlorure cuivrique, le chlorure, le bromure, l'iodure ou le cyanure cuivreux, ou encore un complexe, par exemple l'acétylacétonate de cuivre, le bromure diméthylsulfure cuivreux ou encore le tri-nbutylphosphine chlorure cuivreux ou tout autre complexe du même type connu de l'homme du métier. L'acétate et le proprionate cuivrique sont tout particulièrement préférés.

On opère au sein d'un solvant qui est de préférence un éther tel que le tétrahydrofuranne, le dioxane, le terbutyl-méthyléther, le diméthoxyéthane. Le tétrahydrofuranne est plus particulièrement préféré.

On opère avantageusement à une température de 0 à -30°C et, de préférence, à -20°C.

L'hydrolyse de l'intermédiaire méthylé est de préférence effectuée en versant la solution réactionnelle dans une solution aqueuse d'un phosphate monoalcalin, par exemple de sodium ou de potassium, ou dans un tampon de pH faiblement acide, notamment un tampon phosphate, dans une solution aqueuse de chlorure d'ammonium ou, plus généralement dans un agent acide faible tel que l'acide acétique, propionique ou butyrique.

L'oxydation de l'énol est effectuée de préférence par barbotage d'air dans le milieu d'hydrolyse et l'on opère en présence d'un agent réducteur complexant du cuivre et compatible avec l'oxydant, qui est notamment un dialkylsulfure, le tétrahydrothiophène, un trialkyl ou triphénylphosphite ou la triphénylphosphine. Le diméthyl sulfure et le tétrahydrothiophène sont tout particulièrement préférés. On opère de préférence à température ambiante.

Des procédés de préparation de composés de formule (I) ont déjà été décrits, par exemple dans la demande WO 87/07612. Le procédé de cette demande consiste à méthyler un dérivé 16 insaturé par un agent de méthylation en présence d'un catalyseur à base de cuivre et d'un agent de silylation, dans le but d'isoler intermédiairement un dérivé du type : 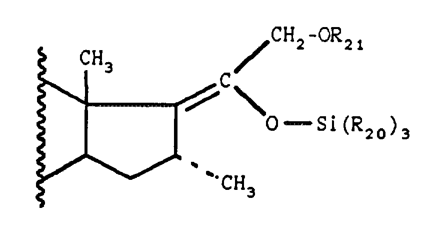 lequel est ensuite traité par un peracide pour former l'époxyde 17α-20 qui est enfin hydrolysé par un acide ou une base.

Le procédé de la présente invention ne requiert l'isolement d'aucun intermédiaire, le produit issu de la réaction de méthylation étant directement hydrolysé puis oxydé dans des conditions jamais envisagées à ce jour et particulièrement intéressantes d'un point de vue industriel. Sa mise en oeuvre est beaucoup plus simple que celle des procédés connus et notamment celle de la demande citée plus haut.

Le brevet anglais 2 001 990 décrit également un procédé de préparation de composés de formule (I) qui consiste comme ci-dessus à méthyler un dérivé 16-insaturé, puis à préparer et isoler l'hydroperoxyde de formule : 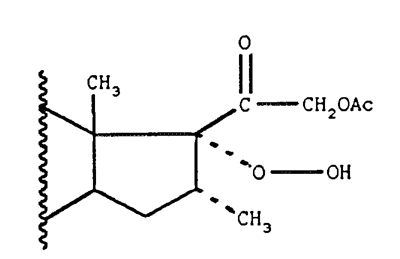 lequel est ensuite réduit par le zinc dans l'acide acétique ou par un iodure alcalin dans une cétone aliphatique. Il convient de noter 1) qu'un tel procédé qui d'abord isole un hydroperoxyde et ensuite le traite par un agent réducteur n'est pas industrialisable en raison des dangers inhérents à ces opérations ; 2) qu'au contraire du procédé de notre demande qui permet d'opérer l'oxydation et la réduction en une seule étape en mettant en oeuvre un réducteur compatible, le procédé ci-dessus ne le permet pas, en raison de l'incompatibilité entre les réactifs qu'il met en oeuvre. En d'autres termes, le principe sur lequel est fondé le procédé de notre demande est différent et ceci tient essentiellement au ligand réducteur (ou oxydable) qu'il utilise et dont la présence est indispensable.

La demande allemande DE-A-2.407.967 décrit un procédé multistade de préparation de composés s'apparentant aux composés de formule (I) et mettant en oeuvre un stéroïde Δ16(17) apparenté aux composés de formule (II), que l'on traite par un réactif de Grignard, le magnésien obtenu étant hydrolysé pour conduire à un dérivé désactivé de type 16α-méthyl pregnan-20-one que l'on traite par une base pour obtenir l'énolate correspondant, que l'on oxyde par l'oxygène pour obtenir un hydropéroxyde qu'ensuite l'on réduit par le zinc dans une étape séparée.

On peut encore citer la référence J. Attenburrow et al J. Chem. Soc. 1961, p. 4547 - 4559, qui décrit un procédé conduisant à des stéroïdes 16β-méthyl, lequel comprend une étape de formation d'un énol en position 20 que l'on oxyde par l'oxygène pour obtenir un hydropéroxyde. Il convient de remarquer que l'énol en question est un composé 16β-méthyl possédant une stabilité particulière qui, selon les auteurs, est probablement due à des facteurs stériques associés à cette configuration.

L'invention a enfin pour objet à titre de produits industriels nouveaux et notamment à titre d'intermédiaires nécessaires à la mise en oeuvre du procédé de l'invention le composé de formule (III) : 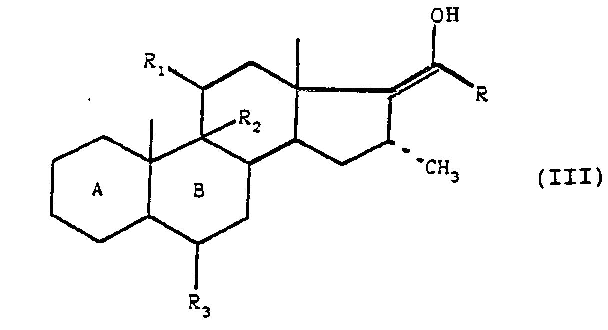 dans laquelle A, B, R, R₁, R₂ et R₃ ont la signification indiquée précédemment et notamment les composés de formule (III) telle que définie ci-dessus dans laquelle les cycles A et B représentent un reste 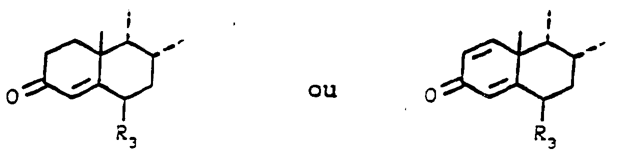 dans laquelle R₃ est défini comme précédemment et la fonction cétone en position 3 est libre et ceux dans lesquels R₁ et R₂ forment ensemble une seconde liaison, ou R₁ et R₂ forment ensemble un époxyde en position β ou R₁ représente une fonction hydroxy en position β libre ou protégée sous forme d'éther ou d'ester et R₂ représente un atome de fluor en position α et R₃ un atome d'hydrogène, un atome de fluor ou un radical méthyle.

Les composés de formule (II) sont connus et décrits, par exemple, dans les brevets US 2 345 711, 2 883 400, 2 963 496, 2 966 504, 2 975 197, 3 029 233, 3 210 341, 3 377 343, 3 839 369, 3 976 638, 4 031 080, 4 277 409, 4 929 395, le brevet allemand 2 207 420, le brevet néerlandais 69 02 507 ou les brevets belges 539 498, 540 478, 711 016, ou peuvent être aisément préparés à partir des composés décrits dans ces brevets, par des procédés connus de l'homme du métier.

Les composés 16α-méthyl de formule (I) sont connus pour posséder une activité anti-inflammatoire et cette formule englobe notamment la dexaméthasone, ses dérivés fluméthasone (6α-fluoro), paraméthasone (6α-fluoro 9H) et des précurseurs possibles (Δ 9,11,9α-OH ou 9,11-époxy).

Les exemples suivants illustrent l'invention sans toutefois la limiter.

### EXEMPLE 1 : 9β, 11β-époxy 16α-méthyl 17α-hydroxy 21-acétyloxy pregna 1,4-diène 3,20-dione

Le mélange à 20°C sous gaz inerte pendant 15 mn, 0,1 g d'acétate cuivrique monohydraté, 3,83 g de 9β, 11β-époxy 21-acétyloxy pregna 1,4,16(17)-triène 3,20-dione et 50 cm³ de tétrahydrofuranne, puis refroidit la solution à -20°C et introduit lentement 3,75 cm³ d'une solution 3 M de bromure de méthylmagnésium dans l'éther éthylique. On agite à -20°C pendant 1 heure.

On refroidit par ailleurs à 0°C environ, 40 cm³ d'une solution aqueuse de phosphate monopotassique à 13,6 % et y ajoute sous agitation en 5 mn environ le mélange obtenu ci-dessus. On laisse remonter la température, sous agitation, de la solution d'énol puis ajoute à +15°C, 4 cm³ de diméthylsulfure en présence d'air.

On fait barboter de l'air dans le mélange, sous agitation, en laissant remonter la température et en condensant le diméthylsulfure entrainé. Après 3 heures, on rajoute 2 cm³ de diméthylsulfure et poursuit le barbotage d'air pendant 2 heures. On décante et extrait à l'acétate d'éthyle. On lave la phase organique à l'eau, sèche et évapore le solvant. On chromatographie le résidu sur silice en éluant au mélange chlorure de méthylène-éther (7-3). On obtient 3,124 g de produit attendu cristallisé.

### Spectre IR (CHCl₃)

Absorptions à 3616 cm⁻¹ (OH), 1747-1728 cm⁻¹ (C=O), 1663-1624-1607 cm⁻¹ (Δ 1,4 3-one).

### Spectre RMN (CDCl₃, 300 MHz, ppm)

0,88 : 16-CH₃ ; 0,93 : H de 18-CH₃ ; 1,44 : H de 19-CH₃ ; 2,16 : CH₃ de OAc ; 2,94 : H de 17-OH ; 3,21 : H en 11 ; 4,69-5,04 : 2H en 21 ; 6,14 : H en 4 ; 6,20 : H en 2 ; 6,62 H en 1.

### Analyse de l'énol intermédiaire

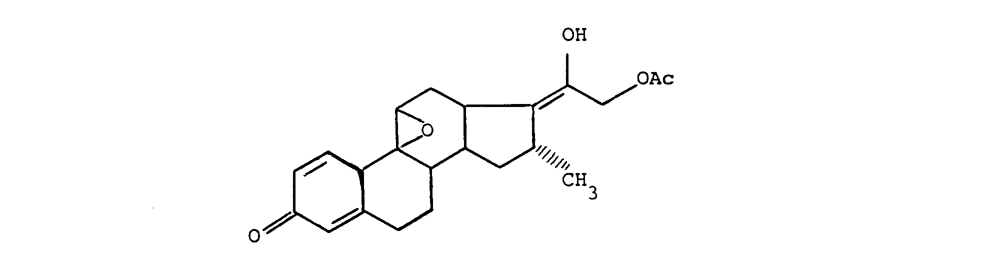

### Spectre IR (CHCl₃) (sous atmosphère inerte)

3588 + associé 3430 cm⁻¹ (F) : OH ; 1738 cm⁻¹ (ep), 1718 cm⁻¹ (max) : >=O ; 1663 cm⁻¹ >=O, 1625 cm-1 C=C, 1607 cm⁻¹ conjugué : Δ₁₋₄ 3-one.

### Spectre RMN (CDCl₃, 400 MHz , ppm)

5,17 et 5,20 (s) isomères E + Z : OH ; 1,01 (d) et 1,08 (d) : CH₃-CH ; 1,08 (s) et 1,10 (s) : 18-Me ; 1,45 : 19-Me ; 2,11 (s) - 2,12 (s) : OAc ; 3,15 et 3,19 : H₁₁ ; 4,49 (d) et 4,67 (d) : C-CH₂O ; 6,16 : H₄ ; 6,20 (dd) : H₂ ; 6,61 (d), 6,63 (d) : H₁.

### Analyse des métaux

Mg 0,1 à 0,2% de la quantité stoéchiométrique théorique nécessaire à la formation d'énolate.

Alcalin 1 à 2% de la quantité stoéchiométrique nécessaire théroique à la formation d'énolate.

### EXEMPLE 2 : 9β, 11β-époxy 16α-méthyl 17a-hydroxy pregna 1,4-diène 3,20-dione

On opère comme indiqué à l'exemple 1, en utilisant au départ 0,02 g d'acétate cuivrique monohydraté et 0,649 g de 9β, 11β-époxy pregna 1,4,16(17)-triène 3,20-dione dans 10 cm³ de tétrahydrofuranne, puis 0,8 cm³ de bromure de méthylmagnésium (3M dans l'éther), 8 cm³ de solution de phosphate monopotassique à 13,6 % et 1,5 cm³ de diméthyl sulfure. On maintient sous agitation et barbotage d'air au total pendant 16 heures. Après extraction à l'acétate d'éthyle, on purifie le produit brut par chromatographie sur silice en éluant au mélange chlorure de méthylène-éther (75-25) et isole 0,326 g de produit attendu cristallisé.

### Spectre IR (CHCl₃)

Absorption à 3610 cm⁻¹ (OH), 1706-1688 cm⁻¹ (C=O), 1663-1625-1607 cm⁻¹ (Δ 1,4 3-one).

### Spectre RMN (CDCl₃, 300 MHz, ppm)

0,88 : 16-CH₃ ; 1,01 (s) : 18-CH₃ ; 1,44 (s) : 19-CH₃ ; 2,24 (s) : -CO-CH₃ ; 3,05 (s) -O-H ; 3,21 (s) : H₁₁ ; 6,15 : H₄ ; 6,18 (dd) : H₂ ; 6,6 (d) : H₁.

### EXEMPLE 3 : 16α-methyl 17α-hydroxy 21-acetyloxy pregna 1,4,9(11)-triène 3,20-dione

On opère comme indiqué à l'exemple 1, en utilisant au départ 0,02 g d'acétate cuivrique monohydraté, 0,733 g de 21-acétyloxy pregna 1,4,9(11), 16(17)-tétraène 3,20-dione dans 10 cm³ de tétrahydrofuranne, puis 0,77 cm³ de bromure de méthylmagnésium (3M dans l'éther), 7 cm³ de solution de phosphate monopotassique à 13,6 % et 2,25 cm³ de diméthylsulfure. On maintient sous agitation et barbotage d'air au total pendant 18 heures puis extrait à l'acétate d'éthyle. On purifie le produit par chromatographie sur silice en éluant au mélange chlorure de méthylène-éther (7-3). On obtient 0,16 g de produit attendu cristallisé.

### Spectre IR (CHCl₃)

Absorption à 3610 cm⁻¹ (OH), 1747-1728 cm⁻¹ (C=O), 1663-1623-1606 cm⁻¹ (Δ 1,4 3-one).

### Spectre RMN (CDCl₃, 300 MHz, ppm)

0,75 : 13-CH₃ ; 0,94 : 16-CH₃ ; 1,40 : 10-CH₃ ; 2,17 : CH₃ de OAC ; 4,82 et 4,99 : 2H en 21 ; 5,54 : H en 11 ; 6,05 : H en 4 ; 6,28 : H en 2 ; 7,19 : H en 1.

### EXEMPLE 4 : 9α-fluoro 11β-hydroxy 16α-méthyl 17α-hydroxy 21-acétyloxy pregna 1,4-diène 3,20-dione

On opère comme indiqué à l'exemple 1, en utilisant au départ 0,02 g d'acétate cuivrique monohydraté et 0,805 g de 9α-fluoro 11β-hydroxy 21-acetyloxy pregna 1,4,16(17) triène- 3,20-dione dans 10 cm³ de tétrahydrofuranne, puis 1,5 cm³ de bromure de méthylmagnésium (3M dans l'éther), 8 cm³ de solution de phosphate monopotassique à 13,6 % et 1,6 cm³ de diméthylsulfure. On maintient sous agitation et barbotage d'air pendant au total 17 heures puis extrait à l'acétate d'éthyle. On purifie le produit par chromatographie sur silice en éluant au mélange chlorure de méthylène-acétate d'éthyle (7-3). On obtient 0,276 g de produit attendu cristallisé.

### Spectre IR (CHCl₃)

Absorptions à 3450-3360 cm⁻¹ (OH), 1740 cm⁻¹ (C=O/acétate), 1720 cm⁻¹ (C=O non conjuguée), 1660 cm⁻¹ (C=O conjuguée).

### Spectre RMN (CDCl₃, 300 MHz, ppm)

0,93 (d) : 16-CH₃ ; 1,07 : 13-CH₃ ; 1,57 : 10-CH₃ ; 2,17 : CH₃ de OAC ; 3,39 : 17α OH et 11β-OH ; 4,38 : 11α-H ; 4,92 : 2H en 21 ; 6,11 : H en 4 ; 6,33 : H en 2 ; 7,25 : H en 1.

### EXEMPLE 5 : 9α-fluoro 11β,21-diacétyloxy 16α-méthyl 17α-hydroxy pregna 1,4-diène 3,20-dione

On opère comme indiqué à l'exemple 1, en utilisant au départ 0,02 g d'acétate cuivrique monohydraté et 0,889 g de 9α-fluoro 11β,21-diacétyloxy pregna 1,4,16(17) triène-3,20-dione dans 10 cm³ de tétrahydrofuranne, puis 0,78 cm³ de bromure de méthylmagnésium (3M dans l'éther), 8 cm³ de solution de phosphate monopotassique à 13,6 % et 1,6 cm³ de diméthylsulfure. On maintient sous agitation et barbotage d'air pendant 18 heures puis extrait à l'acétate d'éthyle. On purifie le produit par chromatographie sur silice en éluant au mélange chlorure de méthylène-éther éthylique (75-25) et obtient 0,634 g de produit attendu cristallisé.

### Spectre IR (CHCl₃)

Absorptions à 3615 cm⁻¹ (OH), 1746 cm⁻¹ (-OAC), 1730 cm⁻¹ (C=O non conjuguée), 1668-1631 et 1610 cm⁻¹ : Δ1,4 3-one.

### Spectre RMN (CDCl₃, 300 MHz, ppm)

0,93 (d) : CH₃ en 16 et 0,93 (s) : CH₃ en 13 ; 1,58 : CH₃ en 10 ; 2,13-2,15 : CH₃ de Ac ; 2,74 : 17α-OH ; 4,71-4,99 : 2H en 21 ; 5,42 : H en 11α ; 6,11 : H en 4 ; 6,33 : H en 2 ; 6,82 : H en 1.

### EXEMPLE 6 : 9β, 11β-époxy 16α-méthyl 17α-hydroxy 21-acétyloxy pregna 1,4-diène 3,20-dione

On opère comme indiqué à l'exemple 1, en utilisant au départ 0,766 g de dérivé 9,11-époxy et 0,02 g d'acétate cuivrique monohydraté dans 10 cm³ de tétrahydrofuranne, 0,8 cm³ de solution 3M de bromure de méthyl magnésium dans l'éther et 8 cm³ de solution tampon phosphate (1 mole d'H₃ PO₄ pour 1,2 mole de soude).

Après 5 minutes sous agitation et gaz inerte à 0°C, on laisse remonter la température à + 18°C, poursuit l'agitation sous barbotage d'air pendant 30 minutes et ajoute 1,2 cm³ de tétrahydrothiophène. On maintient sous agitation et barbotage d'air pendant 4 heures, rajoute 0,4 cm³ de tétrahydrothiophène et poursuit l'agitation et le barbotage. On décante, extrait à l'acétate d'éthyle, lave la phase organique à l'eau, sèche et évapore le solvant. On purifie le produit brut par chromatographie sur silice en éluant au mélange chlorure de méthylène-éther (7-3). On obtient 0,6075 g de produit attendu identique à celui obtenu à l'exemple 1.

## Revendications

1. Procédé de préparation des composés de formule (I) : 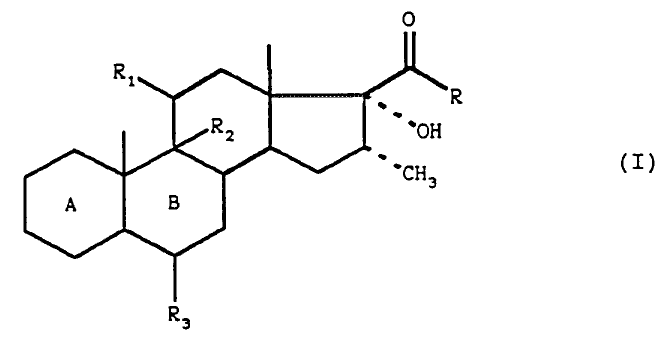 dans laquelle les cycles A et B représentent un reste : 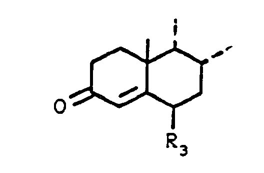 dans lequel la fonction cétone en position 3 est éventuellement protégée sous forme de cétal, de thiocétal, d'hémithiocétal ou d'éther d'énol, ou un reste : 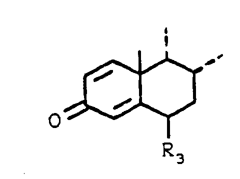 R représente un radical méthyle ou un radical -CH₂-OR', dans lequel R' représente un atome d'hydrogène ou un reste éther ou un reste ester, R₁ et R₂ forment ensemble une seconde liaison, ou R1 et R₂ forment ensemble un époxyde en position β, ou R₁ représente un atome d'hydrogène, une fonction cétone ou une fonction hydroxy en position α ou β, libre ou protégée sous forme d'éther ou d'ester et R₂ représente un atome d'hydrogène, ou R₁ représente un atome d'hydrogène et R₂ représente une fonction hydroxy en position α, ou R₁ représente une fonction hydroxy en position β, libre ou protégée sous forme d'éther ou d'ester et R₂ représente un atome de fluor ou de brome en position α, et R₃ représente un atome d'hydrogène ou un atome de fluor ou un radical méthyle, en position α ou β, caractérisé en ce que l'on traite un composé de formule (II) : 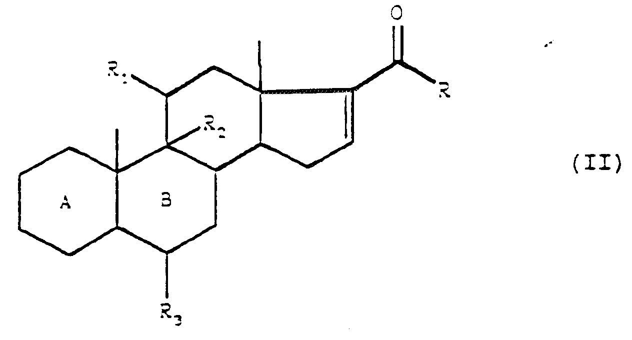 dans laquelle A, B, R, R₁, R₂ et R₃ ont la signification déjà indiquée, par un agent de méthylation en présence d'un catalyseur à base de cuivre, puis hydrolyse l'énolate méthylé pour obtenir l'énol correspondant que l'on oxyde par barbotage d'oxygène ou d'air dans le milieu d'hydrolyse en présence d'un agent réducteur complexant du cuivre et compatible avec l'oxydant, pour obtenir le composé attendu.

2. Procédé selon la revendication 1, caractérisé en ce que l'on utilise au départ un composé de formule (II) dans laquelle les cycles A et B représentent un reste : 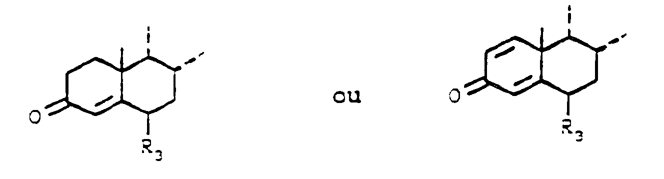 dans lequel R₃ est défini comme à la revendication 1 et la fonction cétone en position 3 est libre.

3. Procédé selon la revendication 1 ou 2, caractérisé en ce que l'on utilise au départ un composé de formule (II) dans laquelle R₁ et R₂ forment ensemble une seconde liaison, ou R₁ et R₂ forment ensemble un époxyde en position β, ou R₁ représente une fonction hydroxy en position β, libre ou protégée sous forme d'éther ou d'ester et R₂ représente un atome de fluor en position α, et R₃ représente un atome d'hydrogène, un atome de fluor ou un radical méthyle.

4. Procédé selon l'une quelconque des revendications 1 à 3, caractérisé en ce que l'on utilise au départ un composé de formule (II) dans laquelle R₃ représente un atome d'hydrogène.

5. Procédé selon l'une quelconque des revendications 1 à,4, caractérisé en ce que l'agent de méthylation est un dérivé méthylé du cuivre ou un chlorure, bromure ou iodure de méthyl magnésium utilisé en présence d'un catalyseur à base de cuivre.

6. Procédé selon l'une quelconque des revendications 1 à 5, caractérisé en ce que l'agent de méthylation est le chlorure, bromure ou iodure de méthyl magnésium utilisé en présence d'acétate ou propionate cuivrique.

7. Procédé selon l'une quelconque des revendications 1 à 6, caractérisé en ce que l'agent d'hydrolyse est choisi dans le groupe constitué par un phosphate monoalcalin, le chlorure d'ammonium, un acide faible et un tampon de pH faiblement acide.

8. Procédé selon l'une quelconque des revendications 1 à 7, caractérisé en ce que l'oxydation d'énol est effectuée par l'air.

9. Procédé selon l'une quelconque des revendications 1 à 8, caractérisé en ce que l'agent réducteur et complexant du cuivre est choisi dans le groupe constitué par les dialkyl-sulfures, le tétrahydrothiophène, les trialkylphosphites, le triphénylphosphite et la triphénylphosphine.

10. Procédé selon l'une quelconque des revendications 1 à 9, caractérisé en ce que l'agent réducteur et complexant du cuivre est choisi dans le groupe constitué par le diméthylsulfure et le tétrahydrothiophène.

11. A titre de produits industriels nouveaux les composés de formule (III) : 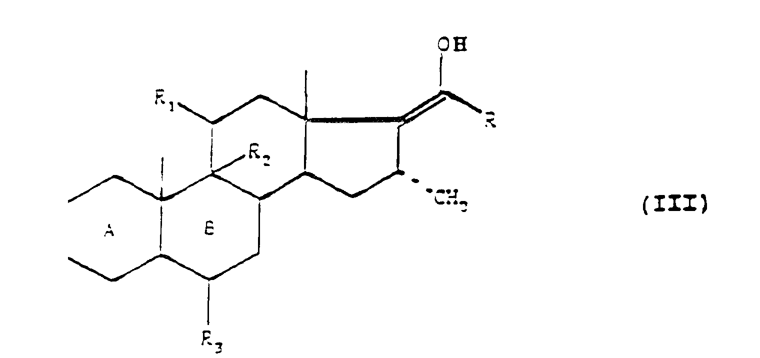 dans laquelle A, B, R, R₁, R₂ et R₃ ont la définition indiquée à la revendication 1.

12. A titre de produits industriels nouveaux les composés de formule (III) définis à la revendication 11 dans laquelle les cycles A et B représentent un reste : 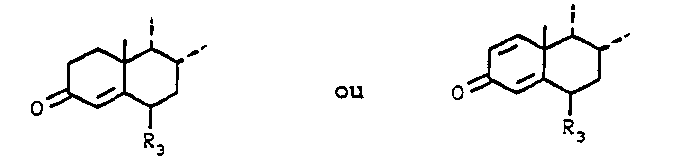 dans lequel R₃ est défini comme à la revendication 1 et la fonction cétone en position 3 est libre et R₁ et R₂ forment ensemble une seconde liaison, ou R₁ et R₂ forment ensemble un époxyde en position β, ou R₁ représente une fonction hydroxy en position β, libre ou protégée sous forme d'éther ou d'ester et R₂ représente un atome de fluor en position α, et R₃ représente un atome d'hydrogène, un atome de fluor ou un radical méthyle.

## Patentansprüche

1. Verfahren zur Herstellung von Verbindungen der Formel (I) 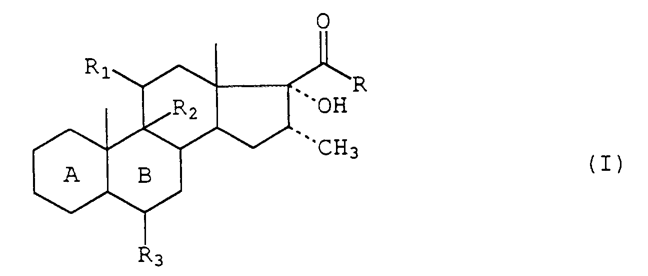 in der die Ringe A und B einen Rest 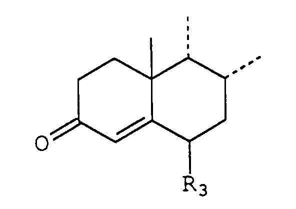 worin die Ketonfunktion in Position 3 gegebenenfalls in Form von Ketal, Thioketal, Hemithioketal oder Enolether geschützt ist, oder einen Rest 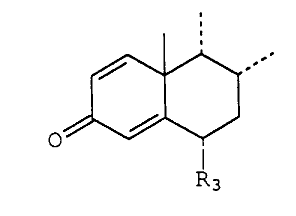 darstellen,
R einen Methylrest oder einen Rest -CH₂-OR' bedeutet, worin R' ein Wasserstoffatom oder einen Etherrest oder einen Esterrest darstellt, R₁ und R₂ zusammen eine zweite Bindung oder ein Epoxid in Position β bilden, oder R₁ ein Wasserstoffatom, eine Ketonfunktion oder eine Hydroxyfunktion in Position α oder β darstellt, frei oder in Form von Ether oder Ester geschützt, und R₂ ein Wasserstoffatom bedeutet, oder R₁ ein Wasserstoffatom darstellt und R₂ eine Hydroxyfunktion in Position α darstellt, oder R₁ eine Hydroxyfunktion in Position β darstellt, frei oder in Form von Ether oder Ester geschützt, und R₂ ein Fluor- oder Bromatom in Position α bedeutet, und R₃ ein Wasserstoffatom oder ein Fluoratom oder einen Methylrest in Position α oder β darstellt, dadurch gekennzeichnet, daß man eine Verbindung der Formel (II) 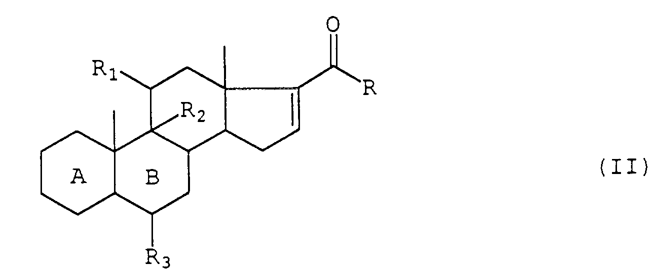 in der A, B, R, R₁, R₂ und R₃ die bereits angegebene Bedeutung besitzen, mit einem Methylierungsmittel in Anwesenheit eines Katalysators auf der Basis von Kupfer behandelt, anschließend das methylierte Enolat hydrolysiert, um das entsprechende Enol zu erhalten, das man durch Einleiten von Sauerstoff oder Luft in das Hydrolysemedium in Anwesenheit eines Reduktionsmittels oxidiert, das mit Kupfer einen Komplex bildet und mit dem Oxidationsmittel kompatibel ist, um die erwartete Verbindung zu erhalten.

2. Verfahren nach Anspruch 1, dadurch gekennzeichnet, daß man als Ausgangsprodukt eine Verbindung der Formel (II) verwendet, in der die Ringe A und B einen Rest 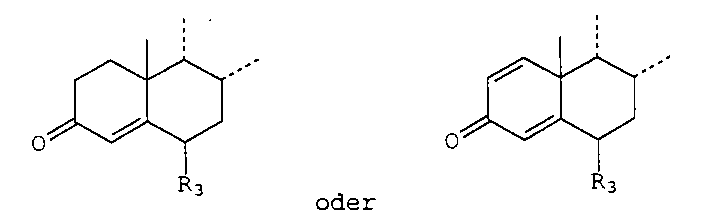 darstellen, worin R₃ wie in Anspruch 1 definiert und die Ketonfunktion in Position 3 frei ist.

3. Verfahren nach Anspruch 1 oder 2, dadurch gekennzeichnet, daß man als Ausgangsprodukt eine Verbindung der Formel (II) verwendet, in der R₁ und R₂ zusammen eine zweite Bindung bilden, oder R₁ und R₂ zusammen ein Epoxid in Position β bilden, oder R₁ eine Hydroxyfunktion in Position β darstellt, frei oder in Form von Ether oder Ester geschützt, und R₂ ein Fluoratom in Position α bedeutet, und R₃ ein Wasserstoffatom, ein Fluoratom oder einen Methylrest darstellt.

4. Verfahren nach irgendeinem der Ansprüche 1 bis 3, dadurch gekennzeichnet, daß man als Ausgangsprodukt eine Verbindung der Formel (II) verwendet, in der R₃ ein Wasserstoffatom darstellt.

5. Verfahren nach irgendeinem der Ansprüche 1 bis 4, dadurch gekennzeichnet, daß das Methylierungsmittel ein Methylderivat von Kupfer oder ein Chlorid, Bromid oder Iodid von Methylmagnesium ist, verwendet in Anwesenheit eines Katalysators auf der Basis von Kupfer.

6. Verfahren nach irgendeinem der Ansprüche 1 bis 5, dadurch gekennzeichnet, daß das Methylierungsmittel ein Chlorid, Bromid oder Iodid von Methylmagnesium ist, verwendet in Anwesenheit von Kupferacetat oder -propionat.

7. Verfahren nach irgendeinem der Ansprüche 1 bis 6, dadurch gekennzeichnet, daß das Hydrolysemittel aus der durch ein Monoalkaliphosphat, Ammoniumchlorid, eine schwache Säure und einen Puffer mit schwach saurem pH-Wert gebildeten Gruppe gewählt wird.

8. Verfahren nach irgendeinem der Ansprüche 1 bis 7, dadurch gekennzeichnet, daß die Enol-Oxidation mit Hilfe von Luft durchgeführt wird.

9. Verfahren nach irgendeinem der Ansprüche 1 bis 8, dadurch gekennzeichnet, daß das Reduktionsmittel, das mit Kupfer einen Komplex bildet, aus der durch die Dialkylsulfide, Tetrahydrothiophen, die Trialkylphosphite, Triphenylphosphit und Triphenylphosphin gebildeten Gruppe gewählt wird.

10. Verfahren nach irgendeinem der Ansprüche 1 bis 9, dadurch gekennzeichnet, daß das Reduktionsmittel, das mit Kupfer einen Komplex bildet, aus der durch Dimethylsulfid und Tetrahydrothiophen gebildeten Gruppe gewählt wird.

11. Als neue industrielle Produkte die Verbindungen der Formel (III) 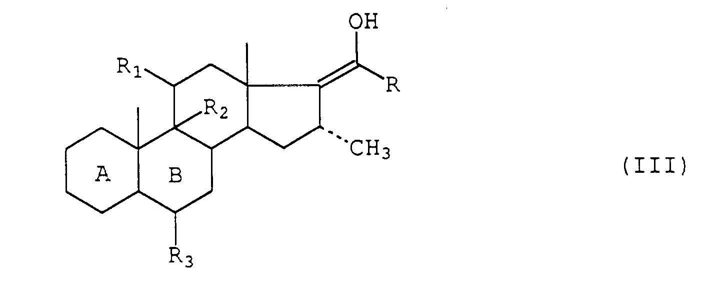 in der A, B, R, R₁, R₂ und R₃ die in Anspruch 1 angegebene Bedeutung besitzen.

12. Als neue industrielle Produkte die Verbindungen der Formel (III) wie in Anspruch 11 definiert, worin die Ringe A und B einen Rest 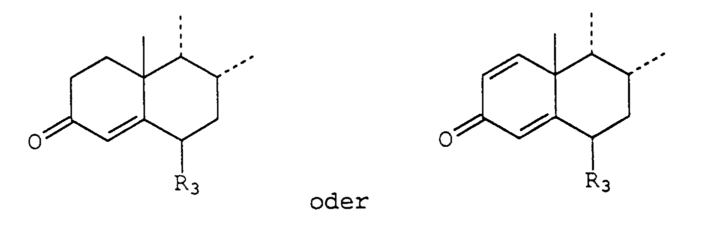 darstellen, worin R₃ wie in Anspruch 1 definiert und die Ketonfunktion in Position 3 frei ist und R₁ und R₂ zusammen eine zweite Bindung bilden, oder R₁ und R₂ zusammen ein Epoxid in Position β bilden, oder R₁ eine Hydroxyfunktion in Position β darstellt, frei oder in Form von Ether oder Ester geschützt, und R₂ ein Fluoratom in Position α bedeutet, und R₃ ein Wasserstoffatom, ein Fluoratom oder einen Methylrest darstellt.

## Claims

1. Preparation process for the compounds of formula (I): 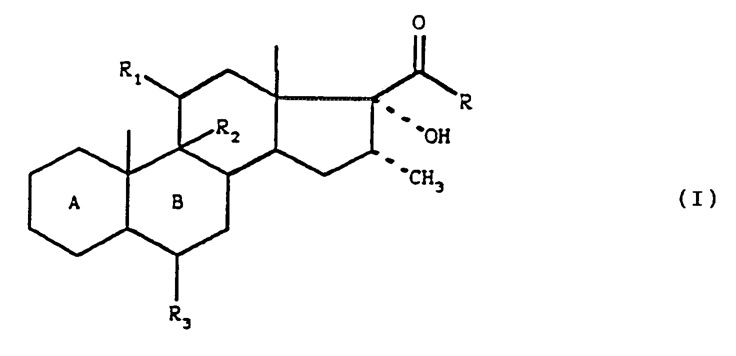 in which the rings A and B represent a remainder: 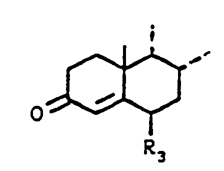 in which the ketone function in position 3 is optionally protected in the form of a ketal, thioketal, hemithioketal or enol ether, or a remainder: 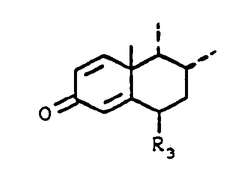 R represents a methyl radical or a -CH₂-OR' radical, in which R' represents a hydrogen atom or an ether remainder or an ester remainder, R₁ and R₂ form together a second bond, or R₁ and R₂ form together an epoxide in position beta, or R₁ represents a hydrogen atom, a ketone function or a hydroxy function in position alpha or beta, free or protected in the form of an ether or ester and R₂ represents a hydrogen atom, or R₁ represents a hydrogen atom and R₂ represents a hydroxy function in position alpha, or R₁ represents a hydroxy function in position beta, free or protected in the form of an ether or ester and R₂ represents a fluorine or bromine atom in position alpha, and R₃ represents a hydrogen atom or a fluorine atom or a methyl radical, in position alpha or beta, characterized in that a compound of formula (II): 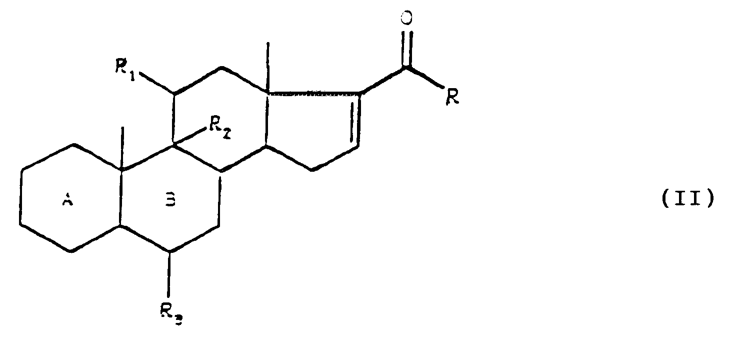 in which A, B, R, R₁, R₂ and R₃ have the meaning already indicated, is treated with a methylation agent in the presence of a copper-based catalyst, then the methylated enolate is hydrolyzed to obtain the corresponding enol which is oxidized by bubbling oxygen or air through the hydrolysis medium in the presence of a reducing and complexing agent of the copper which is compatible with the oxidizing agent, in order to obtain the expected compound.

2. Process according to claim 1, characterized in that at the start a compound of formula (II) is used in which rings A and B represent a remainder: 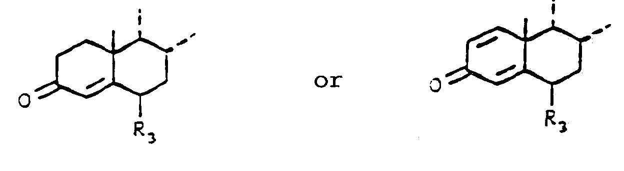 in which R₃ is defined as in claim 1 and the ketone function in position 3 is free.

3. Process according to claim 1 or 2, characterized in that at the start a compound of formula (II) is used in which R₁ and R₂ form together a second bond, or R₁ and R₂ form together an epoxide in position beta, or R₁ represents a hydroxy function in position beta, free or protected in the form of an ether or ester and R₂ represents a fluorine atom in position alpha, and R₃ represents a hydrogen atom, a fluorine atom or a methyl radical.

4. Process according to any one of claims 1 to 3, characterized in that at the start a compound of formula (II) is used in which R₃ represents a hydrogen atom.

5. Process according to any one of claims 1 to 4, characterized in that the methylation agent is a methylated copper derivative or a methyl magnesium chloride, bromide or iodide used in the presence of a copper-based catalyst.

6. Process according to any one of claims 1 to 5, characterized in that the methylation agent is methyl magnesium chloride, bromide or iodide used in the presence of cupric acetate or propionate.

7. Process according to any one of claims 1 to 6, characterized in that the hydrolysis agent is chosen from the group constituted by a monoalkaline phosphate, ammonium chloride, a weak acid and a buffer of weakly acidic pH.

8. Process according to any one of claims 1 to 7, characterized in that the oxidation of enol is carried out using air.

9. Process according to any one of claims 1 to 8, characterized in that the reducing and complexing agent of the copper is chosen from the group constituted by dialkylsulphides, tetrahydrothiophene, trialkylphosphites, triphenylphosphite and triphenylphosphine.

10. Process according to any one of claims 1 to 9, characterized in that the reducing and complexing agent of the copper is chosen from the group constituted by dimethylsulphide and tetrahydrothiophene.

11. As new industrial products, the compounds of formula (III) : 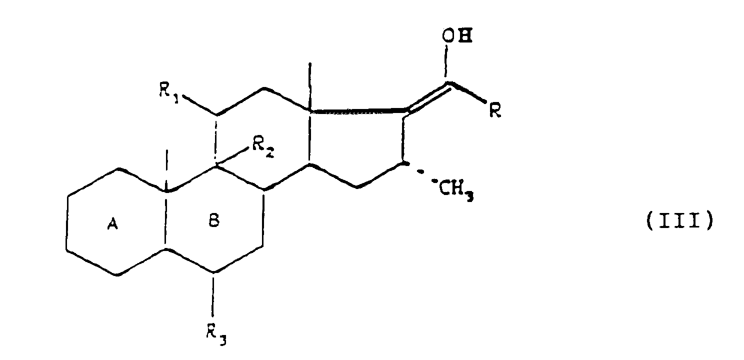 in which A, B, R, R₁, R₂ and R₃ have the definition indicated in claim 1.

12. As new industrial products, the compounds of formula (III) defined in claim 11 in which rings A and B represent a remainder: 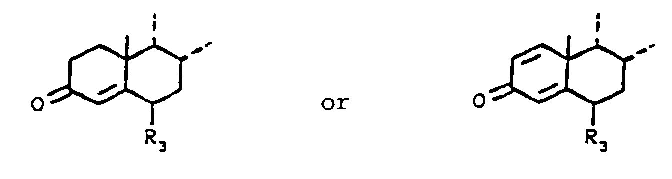 in which R₃ is defined as in claim 1 and the ketone function in position 3 is free and R₁ and R₂ together form a second bond, or R₁ and R₂ together form an epoxide in position beta, or R₁ represents a hydroxy function in position beta, free or protected in ether or ester form and R₂ represents a fluorine atom in position alpha, and R₃ represents a hydrogen atom, a fluorine atom or a methyl radical.
